# FASCICULE DE BREVET EUROPEEN

(11) **EP 1 432 370 B1**
(45) Date de publication et mention de la délivrance du brevet: **17.03.2010**
(21) Numéro de dépôt: 02798760.1
(22) Date de dépôt: 12.09.2002
(51) Int. Cl.: A61F 2/44, A61F 2/46

(54) **PROTHESE DE NUCLEUS INTERVERTEBRAL ET SON DISPOSITIF D'INSERTION**
NUCLEUS-PROTHESE UND EINFÜHRUNGSVORRICHTUNG DAFÜR
NUCLEUS PROSTHESIS AND INSERTION DEVICE THEREFOR

(30) Priorité: 14.09.2001 FR 0111905
(43) Date de publication de la demande: 30.06.2004
(73) Titulaire: Fortin, Frédéric, 33600 Pessac (FR)
(72) Inventeur: FORTIN, Frédéric, F-33600 Pessac (FR); ROBIN, Johann, F-33130 Begles (FR)
(86) Numéro de dépôt international: PCT/FR2002/003103
(87) Numéro de publication internationale: WO 2003/024368

(56) Documents cités:
- EP-A- 0 621 020
- EP-A- 0 919 209
- EP-A- 1 132 061
- WO-A-97/20526
- DE-C- 4 323 595
- US-A- 5 645 597
- US-A- 5 800 549

## Description

**Domaine de l'invention:** La présente invention concerne une prothèse de disque intervertébral et ses moyens d'insertion. Cette prothèse après insertion et enlèvement des moyens d'insertion se substitue au nucléus de la cavité existante après une ablation partielle ou totale de ce dernier en préservant l'annulus du disque intervertébral concerné.

**Art Antérieur :** nous rappelerons brièvement la constitution d'un disque intervertébral liant deux corps vertébraux et qui a un fonctionnement complexe. Ce disque est composé d'un nucléus pulposus situé au centre et d'un annulus fibrosus structure périphérique fibreuse contenant le nucléus et reliant solidement les plateaux vertébraux. Comme toutes les liaisons élastiques du corps humain , les disques intervertébraux sont sujets à la dégénérescence en particulier ceux du rachis lombaire qui sont le plus souvent sollicités.

La plupart des pathologies du disque proviennent d'une fissuration de l'annulus qui laisse échapper une partie du nucléus ce dernier forme alors une protubérance qui se trouve souvent au contact du système nerveux , la zone la plus sensible de l'annulus étant de ce coté . Cette hernis discale vient presser les racines nerveuses ou la dure-mère et provoque des douleurs intenses pouvant aller jusqu'a la paralysie.

Néanmoins, un disque dégénéré peut être traité en remplaçant tout ou partie du nucléus et de l'annulus endommagé. De nombreux systèmes invasifs remplacent la totalité du nucléus ainsi que la majeur partie de l'annulus. Cependant, même s'ils reproduisent les réponses mécaniques appropriées , ils ont tous l'inconvénient d'être très difficiles à ancrer ,obligeant l'opérateur à rajouter des éléments externes à la prothèse qui sont fixés aux vertèbres. Ces pièces métalliques provoquent des réactions de l'organisme qui vont tendre à bloquer les mouvements et la prothèse devient inopérante.

Le brevet EP 0919209 décrit une prothèse de nucléus formée d'un bloc d'hydrogel sec qui après l'implantation va se charger en eau et gonfler d'au moins un millimètre en hauteur l'incision de l'annulus doit dans ce cas être plus large pour pouvoir faire passer le bloc , son rôle de rempart est donc amoindri , les inventeurs ont alors proposés de mettre deux blocs plus petits , chaque bloc étant rentré l'un dans l'autre par un orifice réduit ,dans ce cas les blocs n'ont aucune liaison mécanique et il reste des espaces vides à l'intérieur de la cavité qui ne peut être comblée de façon continue. Des concentrations de contraintes vont apparaître sur l' annulus déjà endommagé , mettant en cause la durée de vie de l'implant et la vitalité de l'annulus.

Le problème peut être résolu à l'aide d'une enveloppe élastique , par un orifice pratiqué dans l'annulus qui vient s'adapter à la cavité par expansion élastique. Il faut ensuite injecter un matériau de comblement visqueux qui en se polymérisant vient combler la prothèse donc remplir la cavité. Cette opération nécessite une phase d'injection supplémentaire qui ,dans certains cas , peut être contraignante du fait qu'elle augmente le temps opératoire.

Le brevet EP 0621 020 A est essentiellement axé sur les moyens d'insertion qui dans ce cas sont *spécifiquement adaptés à des matériaux sphériques de type billes*, et qui ne ressemblent pas aux matériaux à adaptation de forme , de type élastomère qui forment l'essentiel de la présente invention, ce document n'antériorise en aucune façon cette dernière.

le brevet US 5800 549A est un générateur de force pour injecter un implant rachidien , les moyens mis en oeuvre sont différents de ceux de la présente invention qui utilisent des moyens extrêmement simples basés sur le matériau utilisé.

le brevet US 5645 597 A décrit une méthode permettant de retirer le nucléus ,puis une prothèse formée d'un anneau élastique recouvert de deux membranes , l'anneau ayant un trou pour le passage d'un gel introduit par une seringue qui va combler le centre de la cavité prothétique. Dans la présente invention, la prothèse est constituée de deux parties dont chaque partie est parfaitement polymérisée avant d'être introduite séparément, et guidée précisément l'une par rapport à l'autre .

Le brevet EP 1132061A résout un problème différent de celui que l'on cherche à résoudre dans la présente , il s'agit essentiellement dans cette antériorité de faire pousser un greffon sur un os endommagé , les particules injectées sont de l'os non malléable , cela n'a rien à voir avec le problème que veut résoudre la présente demande.

Le document DE 43 23 595 décrit une prothèse de nucleus intervertébral comprenant deux composants structurels s'emboîtant, où le deuxième composant structurel s'insère dans le premier composant structurel plus grand.

La présente invention ne nécessite aucune injection de produits ,elle résout le problème posé avec des moyens complètement différents des antériorités qui n'atteignent en aucune façon la nouveauté et l'activité inventive de la présente invention.

Aucun des documents jusqu'ici examinés n'a essayé de relier les caractéristiques de la prothèse de nucléus aux moyens d'insertions mis en oeuvre .

### DESCRIPTION

Les dessins servant à la compréhension de l'invention sont :

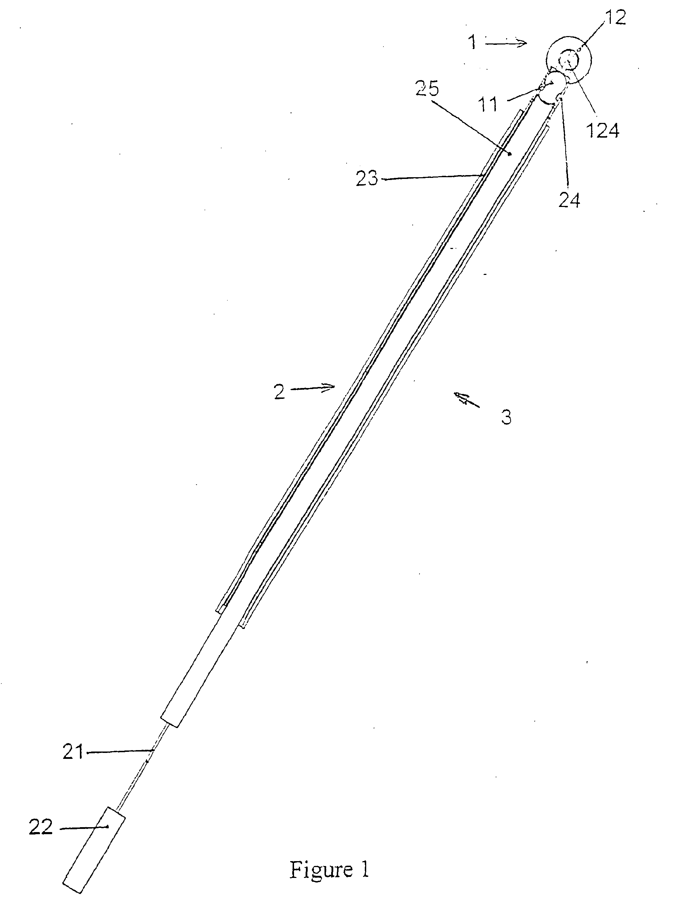

La figure 1 de la planche 1/8 présente une vue en coupe du dispositif complet (prothèse et moyens d'insertion)

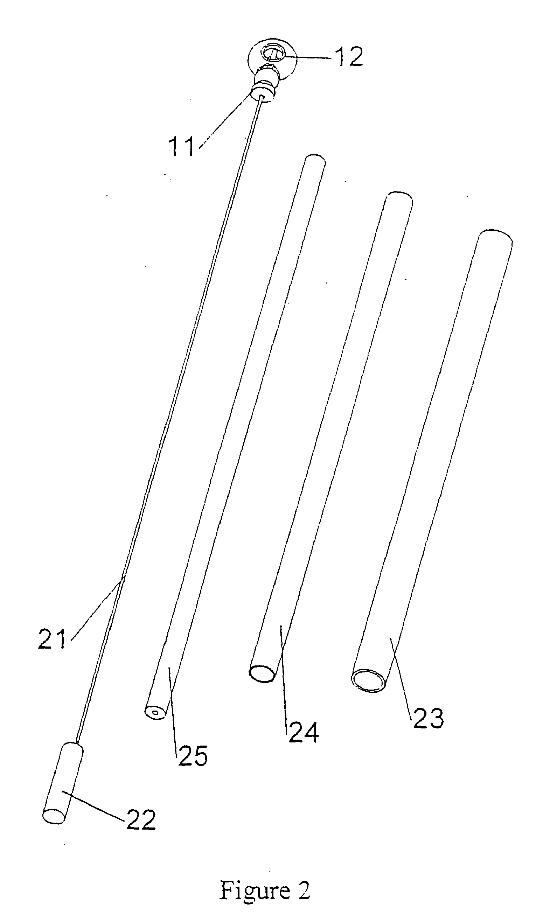

La figure 2 de la planche 2/8 est une vue éclatée des moyens qui composent l'invention.

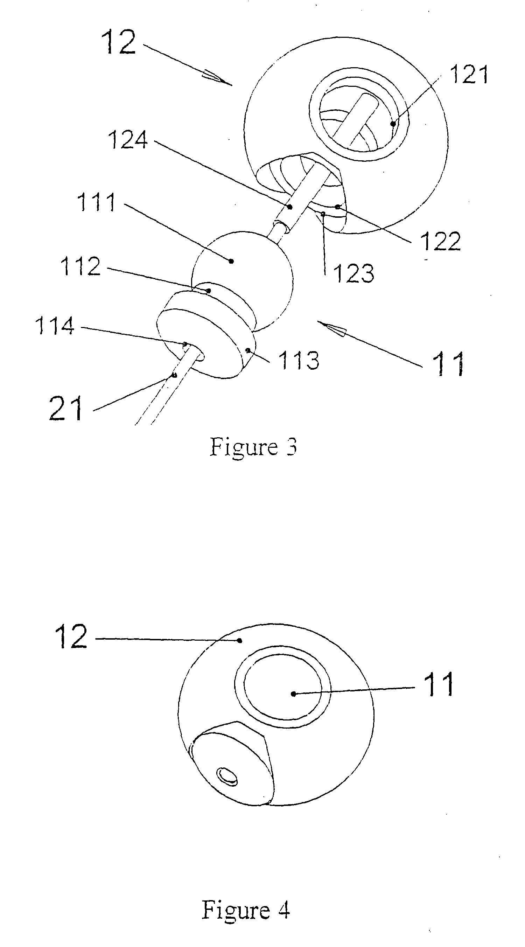

La figure 3 de la planche 3/8 est une vue en perspective de la prothèse formée de deux parties distinctes.
La figure 4 de la planche 3/8 est une vue en perspective de la prothèse apres réunion des 2 parties.

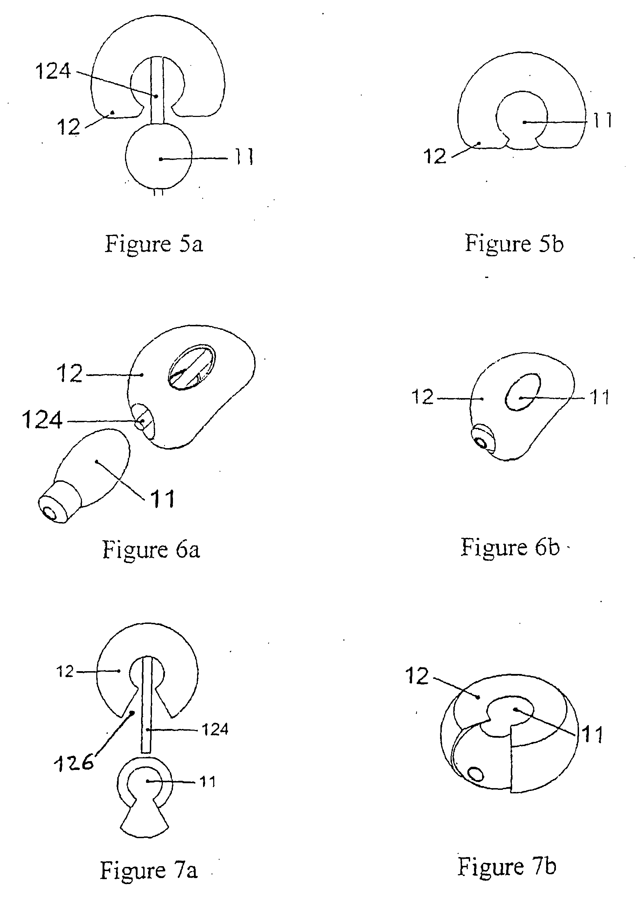

La figure 5a de la planche 4/8 est une vue en coupe horizontale de la prothèse formée de ses 2 parties avant assemblage
La figure 5b de planche 4/8 est une vue en coupe horizontale de la prothèse formée de ses 2 parties réunies
La figure 6a de la planche 4/8 montre une vue en perspective d'une prothèse de forme différente à l'origine.
La figure 6b de la planche 4/8 est la même prothèse assemblée la partie mâle ayant été introduite dans la partie femelle.
Les figures 7a et 7b de la planche 4/8 représentent les mêmes situations que les figures 6a et b, la partie femelle étant dans ce cas un anneau fendu sectoriellement de part en part.
La figure 8 de la planche 5/8 montre l'ensemble des moyens qui participent à l'invention avant l'introduction dans le disque intervertébral.
La figure 9 de la planche 5/8 montre l'ensemble des moyens après introduction dans ledisque intervertébral de la partie femelle.
La figure 10 de la planche 5/8 est la même prothèse que celle de la figure 9 ,mais après introduction de la partie mâle dans la partie femelle.
La figure 11 de la planche 5/8 représente la même prothèse une fois retirés certains moyens servant à l'insertion.
La figure 12 de la planche 5/8 montre la prothèse une fois en place , les tiges de guidage ayant èté coupées
La figure 13 de la planche 5/8 montre une vue en coupe longitudinale de la prothèse plaçée dans le disque intervertébral.
Les figures 14 ,15 et 16 de la planche 6/8 montrent une prothèse dans laquelle la partie mâle est une variante qui permet une fixation sur l'os

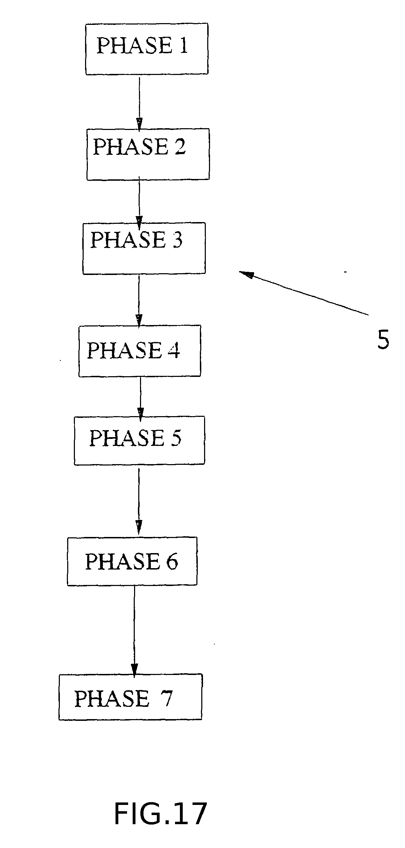

La figure 17 de la planche 7/8 donne les phases du procédé de mise en place.
Les figures 18 et 19 de la planche 8/8 sont des vues en perpective de l'ensemble d'une autre réalisation de la prothèse montrant les détails avant insertion.
La figure 20 de la planche 8/8 est une vue en coupe (en forme de bouchon de champagne) d'une prothèse partie mâle et femelle en place
La figure 21 de la planche 8/8 montre la même prothèse une fois enlevées les tiges de guidage
La figure 22 de la planche 8/8 est une vue en coupe verticale de la prothèse.
La figure 23 de la planche 8/8 est une vue en coupe verticale de la prothèse .implantée

La prothèse de nucléus intervertébral (1) comprend dans une première forme de réalisation
un moyen 12 appellé partie femelle , fabriqué en matériau défor-mable élastiquement dont la forme peut être une sphère creuse aux pôles aplaties, formée:
d'une cavité centrale 121 reliée à une tige rigide 21 et
d'un orifice d'introduction 122
un moyen 11 partie mâle qui comporte une sphére pleine 111 déformable dont les fonctions principales sont:
d'écarter la partie femelle 12 pour s'introduire et venir épouser parfaitement la forme de la cavité 121 apres introduction
d'être apte à supporter les efforts mécaniques imposés .

Ce moyen 11 possède dans sa partie arrière une excroissance 113 dont la forme est déterminée pour venir s'encastrer dans l'ouverture 122 qui s'écarte par élasticité pendant l'introduction. Cette opération effectuée , la partie mâle 11 prend sa place de manière définitive et ne peut être expulsée de la partie femelle 12.

La prothèse de nucléus 1 est introduite dans la cavité intervertébrale par un ensemble de moyens d'insertion 2 permettant son introduction , à savoir:
la tige rigide 21 reliée au moyen 12 par l'intermédiaire d'une liaison souple 124
des tubes 23 ,24 et 25 servant à introduire le moyen 1 dans la cavité.Le tube 23 contient la partie femelle 12 de la prothèse 1 comprimée avec sa tige de guidage 21; il contient également le tube 24.

Le tube 24 contient la partie mâle 11 guidée par la tige 21 , ce moyen 24 servira de poussoir à la partie femelle 12 comprimée à l'intérieur du tube 23.

Le tube 25 enfilé sur la tige 21 sert de poussoir à la partie mâle 11 vers la partie femelle 12.

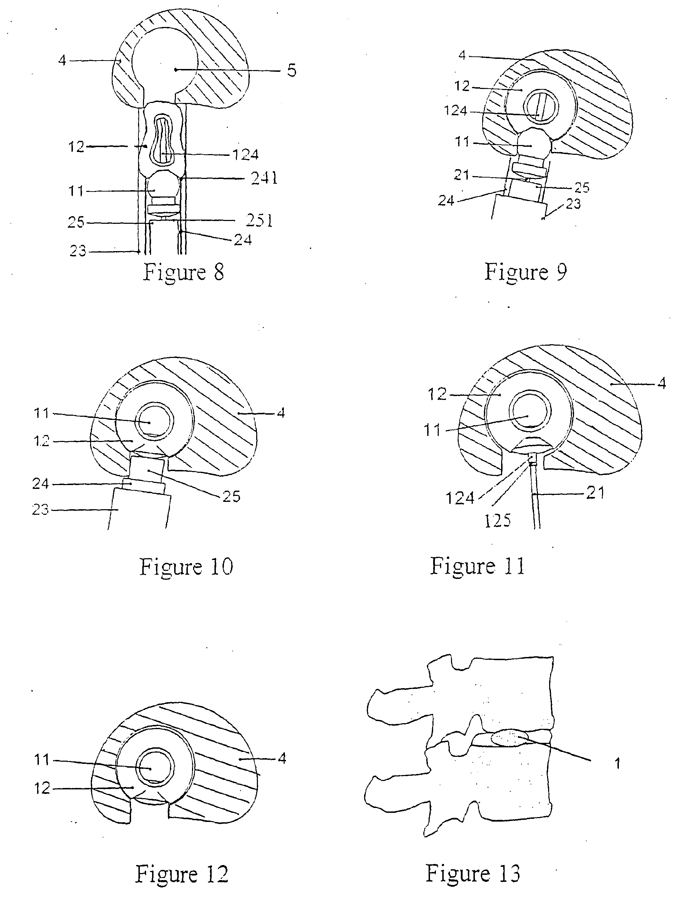

Le Procédé d'insertion 5 comprend 7 phases
phase 1 introduction dans le tube 24 de la partie mâle 11 enfilée sur la tige 21
phase 2 coulissement du tube 23 autour du tube 24, la partie femelle 12 est introduite dans le tube 23
phase 3: présentation du dispositif d'insertion 1 devant l'orifice de la cavité à combler
phase 4 poussée de la partie femelle 12 par le tube 24 (fig 9)
phase 5 introduction du tube 25 enfilé sur la tige 21 pour amener la partie mâle 11 dans l'orifice 122 de la partie femelle 121
phase 6 poussée à l'aide du tube 25 de la partie mâle 11 dans l'orifice 122 pour encastrer le moyen 11 dans le moyen 12.
phase 7 les tubes 23,24,25 sont retirés , la tige 21 étant enlevée ,par exemple par coupure au niveau de la liaison souple 124, ce qui libère la prothèse 1 du dispositif d'insertion 2.Les formes des prothèses de nucléus 1 pièces mâles 11 et femelle 12 peuvent avoir plusieurs variantes , toujours compatibles avec le même procédé d'insertion 5.

La partie femelle 12 a un orifice 122 qui peut être soit une forme circulaire soit la forme d'une fente traversant sectoriellement la prothèse 12 de part en part qui se présente comme un anneau fendu.

Il existe toujours dans cet orifice 122 un rétrécissement 123 qui va interdire à la partie mâle 11 d'être explusée une fois positionnée dans la partie femelle 12

Les formes extérieures de l'enveloppe non contraintes peuvent varier , la fonction essentielle étant de s'adapter à la cavité intervertébrale à combler sans risque de glissement ou d'expulsion.

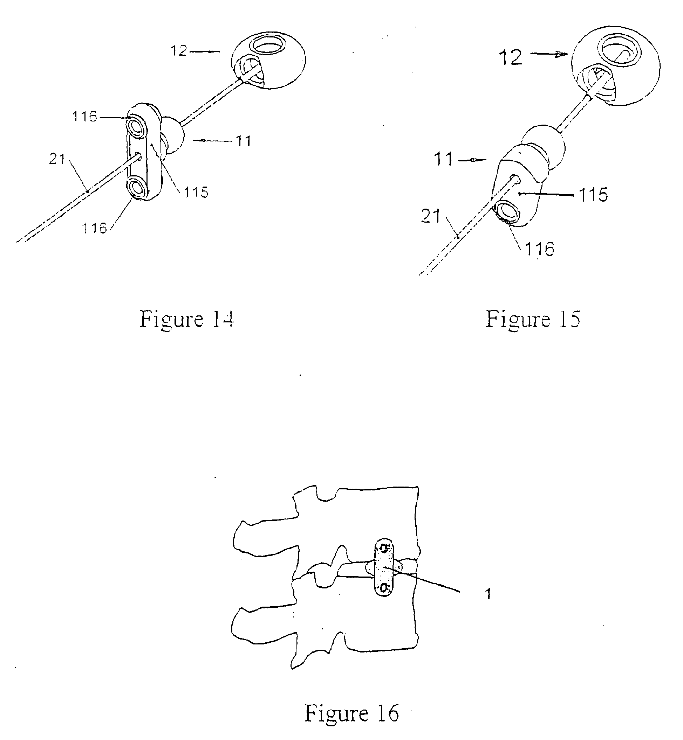

La partie mâle 11 ( fig 14 et 15) peut comporter à son extrémité une excroissance 115 qui comporte une fixation 116 sur les os exemple : des trous laissant passer des vis de fixation , ceci afin de mieux garantir dans certains cas la non explusion de la prothèse.La partie femelle peut grâce à son adaptation de forme provenant de la nature de son matériau élastique a une capacité de déformation qui lui permet de passer au travers des tubes et orifices d'insertion qui ont des sections plus réduites, on peut apprécier le taux de réduction ou compression (rapport R entre les diamètres D et d avant et après mise en place dans le tube ) *R est supérieur à 2*

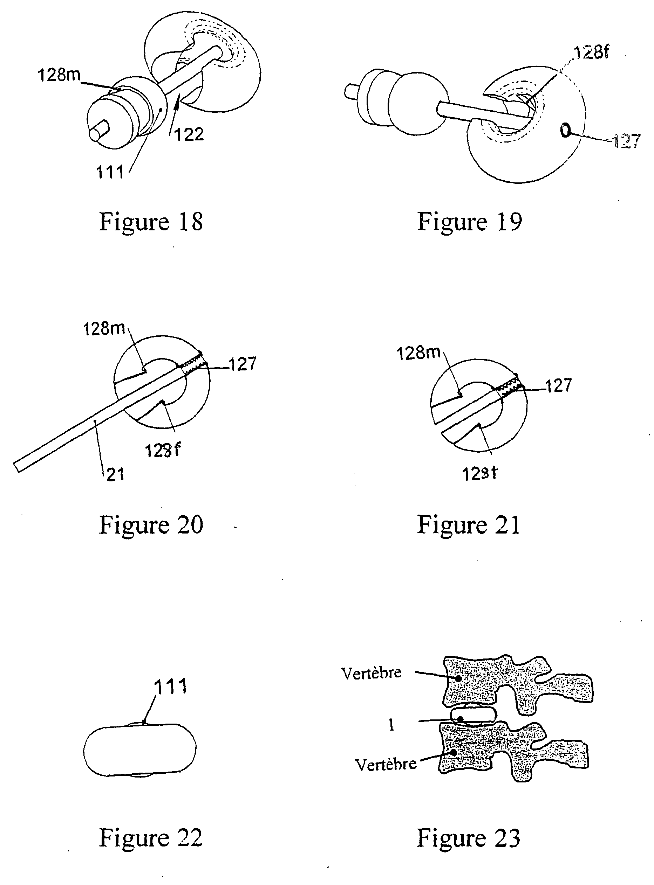

Dans une autre forme de réalisation ( voir figures 18, 19,20 21 et 22) la prothèse de nucléus 1 déformable élastiquement comprend deux parties ;
un moyen 12 appelé partie femelle ayant une forme d'anneau ouvert pour laisser rentrer une partie mâle 11, le moyen 12 comporte un filetage 127f recevant une tige de guidage 21 dont l'extrémité est filetée (filetage mâle 127m correspondant à 127f ), ladite tige rigide 21 permet de guider librement la partie mâle 11de la prothèse 1 jusqu'à son insertion et également de retirer la tige 21 par simple dévissage, une fois la prothèse 1 en place.

Le moyen 12 comprend un orifice 122 permettant l'introduction du moyen 11 (partie mâle 127a). Le moyen 11 a de préférence la forme d'un bouchon antiretour , il comporte une tête sphérique pleine 111 déformable ainsi qu'un corps cylindrique dépassant légèrement si et se trouvant à la périphérie de l'anneau.

Dans cette solution , l'ancrage de la partie mâle 11 dans la partie femelle est assuré par un système anti-retour de clipsage des parties mâle 128m et femelle 128f , système intégré dans la forme des pièces lors de la fabrication.

Lors de l'introduction , la partie mâle 11 écarte l'orifice 122 de l'anneau, ce dernier par élasticité vient ensuite se refermer sur la partie mâle 11. De plus (voir figure 22) la tête sphérique de la partie male dépasse légèrement de l'épaisseur de l'anneau . Quand la prothèse est soumise aux sollicitations dynamiques, cette partie sphérique est comprimée la première, elle augmente ainsi la fonction antiretour rendant impossible l'expulsion de la partie male.

La partie femelle peut disposer d'un insert métallique recevant la tige filetée 127m qui est visible sous rayons X .Une fois la prothèse 1 en place ,la position de l'insert permet de vérifier la stabilité de la prothèse dans le temps, il permet également un démontage facile de la tige 21 une fois la prothèse installée.

L'invention n'est pas limitée aux exemples décrits et représentés car diverses modifications peuvent y être apportées sans sortir de son cadre, defini par les revendications suivantes.

## Revendications

1. Prothèse de nucléus intervertébral (1) composée de deux parités fabriquées dans un matériau élastique déformable, **caractérisée en ce qu'**elle comprend:
- une partie femelle (12) déformable comprenant une cavité centrale (121) apte à être reliée à une tige rigide (21), la cavité centrale (121) comportant un orifice (122) lui même déformable;
- une partie mâle (11) comprenant une sphère pleine (111) déformable élastiquement en vue de venir se loger pour épouser la forme de la cavité centrale (121) grâce à l'orifice déformable (122), ceci pour former un bloc élastique non expulsable de son logement quand la prothèse est soumise aux efforts mécaniques imposes.

2. Prothèse de nucléus intervertébral (1) selon la revendication 1, **caractérisée en ce que** la partie femelle (12) a la forme d'une sphère creuse aplatie aux pôles, et **en ce que** ledit orifice (122) a une forme circulaire, l'orifice possédant un rétrécissement (123) évitant l'expulsion après introduction de la partie mâle (11).

3. Prothèse de nucléus intervertébral (1) selon la revendication 1, **caractérisée en ce que** la partie femelle (12) a la forme d'une sphère creuse aplatie aux pôles, et **en ce que** ledit orifice (122) est une fente (126) traversant de part en part sectoriellement l'épaisseur de la partie femelle (12) et possédant un rétrécissement (123) évitant l'expulsion après introduction de la partie mâle (11).

4. Prothèse de nucléus intervertébral (1) selon l'une quelconque des précédentes revendications 2 ou 3 **caractérisée en ce que** la partis mâle (11) comporte une excroissance (115) à son extrémité qui comporte une fixation (116) permettant le passage de vis pour fixation sur l'os pour assurer un bon ancrage de la prothèse (1).

5. Prothèse de nucléus intervertébral (1) selon la revendication 1, **caractérisée en ce que** la partie femelle (12) a une forme d'anneau ouvert pour laisser rentrer la partie mâle (11), ladite partie femelle (12) comportant un filetage (127f) pour recevoir une tige de guidage (21) dont l'extrémité est filetée.

6. Prothèse de nucléus intervertébral (1) selon la revendication 5, **caractérisée en ce que** la partie mâle a la forme d'un bouchon de champagne jouant le rôle d'anti-retour comportant une tête sphérique pleine (111) déformable ainsi qu'un corps cylindrique dépassant légèrement et se trouvant à la périphérie de l'anneau.

7. Prothèse de nucléus intervertébral (1) selon l'une quelconque des précédentes revendications 1 à 6, **caractérisée en ce que** la partie femelle (12) possède un insert qui permet sous visualisation de rayonnement X de vérifier la stabilité de ladite prothèse dans la temps ainsi que le démontage de la tige (21) une fois la prothèse installée.

8. Combinaison d'une prothèse selon l'une quelconque des précédentes revendications 1 à 4, et d'un dispositif (3) de mise en place de ladite prothèse (1) **caractérisée en ce que** les moyens d'insertion (2) du dispositif (3) sont formés d'une tige (21) rigide reliée à la partie femelle (12) par une liaison souple (124) et de tubes (23, 24, et 25) servant à introduire la prothèse (1) de nucléus dans la cavité intervertébrale à combler.

9. Combinaison d'une prothèse selon l'une quelconque des précédentes revendications 5 ou 6, et d'un dispositif (3) de mise en place de ladite prothèse (1) **caractérisée en ce que** les moyens d'insertion (2) du dispositif (3) comprennent une tige (21) rigide dont l'extrémité a un filetage mâle (127m) correspondent à le filetage (127f) de la partie femelle (12), ladite tige rigide (21) permettant également de guider librement la partie mâle (11) de la prothèse (1) jusqu'à son insertion et ensuite de pouvoir retirer la tige (21) par simple dévissage, une fois la prothèse (1) en place.

## Claims

1. Intervertebral nucleus prosthesis (1) composed of two parts made of elastic deformable material, **characterized in that** it comprises:
- a deformable female part (12) comprising a central cavity (121) capable of being connected to a rigid rod (21), this female part including a deformable orifice (122)
- a male part (11) comprising a elastically deformable full sphere (111) to come and position itself in matching the shape of the central cavity (121), this, thanks to the deformable orifice (122), forms an elastic block which cannot be expelled from its housing, when the prosthesis is subjected to imposed mechanical forces.

2. Intervertebral nucleus prosthesis (1) according to claim 1 **characterized in that** the female part (12) has a shape of hollow sphere flattened at the poles, and **in that** the orifice (122) has a circular shape, and presents a constriction (123) avoiding the expulsion after introduction of the male part (11).

3. Intervertebral nucleus prosthesis (1) according to claim 1 **characterized in that** the female part (12) has a shape of a hollow sphere flattened at the poles, and that the deformable orifice (122) cuts a sector (126) through the thickness of the female part (12) having a constriction (123) which prevents from the expulsion of the male part (11) after its introduction.

4. Intervertebral nucleus prosthesis (1) according to any of previous claims 2 or 3 **characterized in that** the male part (11) has a excrescence (115 ) at its extremity comprising a fixation (116) allowing a screw insertion into the bone to better ensure a good anchorage of the prosthesis (1).

5. Intervertebral nucleus prosthesis (1) according to claim 1 **characterized in that** the female part (12) have a shape of opened ring, allowing to let enter the male part (11) inside , and that the female part (12) comprises a thread (127f) for receiving the guidance rod (21) whose end has a corresponding thread.

6. Intervertébral nucleus prosthesis (1) according to claim 5 **characterized in that** the male part (11) has a shape of champagne cork, serving as a ane way device comprising a deformable fully spherical head (111) and a slightly extending cylindrical body at the ring periphery.

7. Intervertebral nucleus prosthesis (1) according to any of previous claims 1 to 6 **characterized in that** the female part (12) has an insert which allows under x-ray control to check the stability of the prosthesis (1) at any time, and the disassembly of the guidance rod (21), once the prosthesis is in place.

8. Combination of a prosthetis (1) and its introduction device (3) according to any of previous claims 1 to 4 **characterized in that** the insertion means (2) of the device (3) are composed of a rigid rod (21) linked to the female part (12) by a flexible link (124) and by tubes (23, 24, 25) to fill the intervertebral cavity with the prosthesis (1).

9. Combination of a prosthetis (1) and his introduction device (3) according to any of previous claims 5 or 6 **characterized in that** the insertion means (2) of the device (3) include a rigid rod (21) whose end has a male thread (127m) in connection with the female thread (127f) of the female part (12), said rigid rod (21) allowing to freely guide the male part (11) of the prosthesis (1) until its insertion, and then remove the rod (21), by unscrewing it, once the prosthesis (1) is in place.

## Patentansprüche

1. Prothetik intervertebrale kern (1) bestehend aus zwei teilen im elastischen material verformbaren **gekennzeichnet**, dass es auch :
- eine weibliche teil (12) verformbar bestehend aus einem zentralen hohlraum (121) an einem starren Stab verbunden (21), den zentralen Hohlraum (121) aus einer Öffnung (122) sich verformbare elastisch verformbare
- einen männlichen teil (11) mit einem festen kugel (111) um schutz durch zu können in passender form der höhle bleiben (121) durch die Öffnung (122), diese bilden eine elastische block, nicht geräumt, wenn die Prothese, mechanische kräfte beschränkungen unterworfen ist.

2. Prothetik intervertebrale kern (1) nach ansprüche 1 **gekennzeichnet** in diesem den weiblichen teil (12) hat einer rundschreibenform hohle kugel abgeflacht, und was der blende(122) hat eine runde, die blende (122) mit verengung (123) vermeidung von vertreibung nach einführung des männlichen (11).

3. Prothetik intervertebrale kern (1) nach ansprüche 1 **gekennzeichnet** in diesem weibliche teil (12) hat die form einer hohlkugel abgeflacht an den polen und in diesem die blende (122) ist eine schlitz (126) vollständig durch und sektoral dicke von weibliche teil (12) mit verengung (123) vermeidung von vertreibung nach einführung des männlichen (11).

4. Prothetik intervertebrale kern (1) nach beliebig zurüch ansprüche 2 oder 3 **gekennzeichnet** in diesen, der männlichen teil (11) umfasst einen vorsprung (115) bis zum ende, mit einer fixierung (116) durch einleiten von schrauben zur fixierung von knochen, für eine gute verankerung der prothetik (1).

5. Prothetik intervertebrale kern (1) nach ansprüche 1 **gekennzeichnet** in diesem der weibliche teil (12) hat eine eröffnet ringförmige oslassen der männlichen (11), der weibliche teil (12) mit einem gewinde (127f) die entgegennahme einer führungsstange (21) deren ende mit gewinde.

6. Prothetik intervertebrale kern (1) nach ansprüche 5 **gekennzeichnet** in diesem der männlichen teil (11) hat die form eines champagner-korken als ein antirückker mit einem kugelförmigen verformbaren kopf (111) und einem zylindrischen körper etwas mehr als platziert an der peripherie des ringes.

7. Prothetik intervertebrale kern (1) nach beliebig zurüch ansprüche 1 zu 6 **gekennzeichnet** in diesen, den weiblichen teil (12) hat eine einlage dass die pe, die Visualisierung ermöglicht unter x-ray, die zur zeit der Stabilität der prothese (1) und sobald die prothese, die demontage des Schaftes (21).

8. Kombination aus einer prothetik intervertebrale kern (1) und eine vorrichtung (3) nach beliebig zurüch ansprüche 1 zu 4 **gekennzeichnet** in diesem die einfügung mittel (2) der vorrichtung (3) gehören: ein Stamm (21) auf den weiblichen Teil (12) durch eine flexible verbindung (124 verbunden und rohre (23) (24) und (25) zum zuführen der prothetik intervertebrale kern (1) in die kavität intervertébrale.

9. Kombination aus einer prothetik intervertebrale kern (1) und eine vorrichtung (3) nach beliebig zurüch ansprüche 5 oder 6 **gekennzeichnet** in diesem die einfügung mittel (2) der vorrichtung (3) gehören ein Stamm (21) dessen einem ende ein außengewinde (127m) entsprechend dem thread (127f) der weiblichen Teil (12), das sagte starren Stab (21) frei führer der männlichen (11) von der prothetik intervertebrale kern (1) der integration von macht und entfernen sie dann den stamm (21) einfach durch abschrauben, sobald der prothetik intervertebrale kern (1) bis.
